# FASCICULE DE BREVET EUROPEEN

(11) **EP 2 233 560 B1**
(45) Date de publication et mention de la délivrance du brevet: **03.04.2019**
(21) Numéro de dépôt: 10305161.1
(22) Date de dépôt: 17.02.2010
(51) Int. Cl.: C12M 1/34

(54) **Dispositif de culture cellulaire ou tissulaire et ensemble associé.**
Vorrichtung zur Zell- oder Gewebekultur und entsprechende Anordnung
cell or tissue culture device and associated assembly

(30) Priorité: 18.02.2009 FR 0951062; 03.04.2009 US 166303 P
(43) Date de publication de la demande: 29.09.2010
(73) Titulaire: L'Oréal, 75008 Paris (FR)
(72) Inventeur: Azadiguian, Gayané, 75012, PARIS (FR); Carlier, Frédéric, 91390, MORSANG SUR ORGE (FR); Black, Annie, 92300, LEVALLOIS PERRET (FR); Boyera, Nathalie, 69003, LYON (FR); Emmerechts, Carl, 4500, HUY (BE)
(74) Mandataire: Lavoix

(56) Documents cités:
- EP-A- 0 688 602
- WO-A-94/28111
- WO-A-2004/020571
- WO-A-2006/131123
- US-A- 5 578 492
- US-A- 5 801 055
- US-A1- 2001 036 426

## Description

La présente invention concerne un dispositif de culture cellulaire ou tissulaire.

Un tel dispositif est destiné notamment à la culture in vitro de différents types de cellules et/ou de tissus, en vue d'utiliser ces cultures pour différents tests ou évaluations. Les échantillons de culture cellulaire s'étendent généralement dans deux dimensions, alors que les échantillons de culture tissulaire s'étendent dans trois dimensions.

Un tel dispositif est notamment destiné à produire des échantillons de tissus de cellules cutanées, tels que des échantillons de peau reconstruite.

On connaît de WO 2006/131123 un dispositif du type précité, destiné à être monté sur un bac contenant un milieu de culture liquide.

Le dispositif comprend un support monté sur le bac, et une pluralité de nacelles amovibles présentant chacune un substrat perméable pour la culture de cellules ou de tissus. La culture des cellules est effectuée en disposant les cellules sur la surface de culture et en alimentant ces cellules à travers le substrat grâce au milieu de culture présent dans le bac inférieur.

A cet effet, les nacelles sont disposées sur le support dans une position de culture. Le support et les nacelles sont ensuite montés conjointement sur le bac pour imprégner le substrat perméable de chaque nacelle par le milieu de culture et permettre ainsi la croissance des cellules.

Chaque nacelle étant mobile individuellement par rapport au support, il est possible de charger et/ou de décharger successivement chaque nacelle de manière indépendante, sans avoir à retirer les autres nacelles du support ou du milieu de culture.

Un tel dispositif ne donne pas entière satisfaction, notamment pour réaliser des opérations de culture ou de test complexes qui nécessitent parfois de retourner simultanément toutes les nacelles, de mettre en contact simultanément tous les échantillons à l'interface air-liquide ou en contact avec un réactif, ou d'effectuer un traitement thermique simultané de tous les échantillons.

En outre, lorsque les échantillons de culture doivent être agités, le dispositif décrit dans WO 2006/131123 n'est pas très robuste et peut conduire au décrochement des nacelles avec perte des échantillons qu'elles contiennent.

EP 0 688 602 décrit un système de microtitration destiné à être utilisé dans le domaine du diagnostic. Ce système ne comprend pas de bac contenant un milieu de culture, ni de nacelle délimitant un passage d'alimentation d'une surface de culture en moyen de culture, le passage étant distinct d'une ouverture supérieure de la nacelle.

US 2001/0036426 décrit un dispositif de stockage d'échantillons configuré pour éviter toute contamination d'un échantillon contenu sur une plaque au moyen d'un bouchon fixé sur l'échantillon. Ce dispositif ne comprend pas de nacelles mobiles.

Un but de l'invention est d'obtenir un dispositif de culture permettant de cultiver individuellement différents échantillons de cellules ou de tissus et qui présente néanmoins une manipulation aisée, notamment lors de différentes opérations nécessaires à la culture ou à des tests complexes.

A cet effet, l'invention a pour objet un dispositif selon la revendication 1.

Le dispositif selon l'invention peut comprendre l'une ou plusieurs des caractéristiques des revendications 2 à 12.

L'invention a également pour objet un ensemble de culture cellulaire ou tissulaire, selon la revendication 13.

L'ensemble selon l'invention peut comprendre l'une ou plusieurs des caractéristiques des revendication 14 et 15.

L'invention sera mieux comprise à la lecture de la description qui va suivre, donnée uniquement à titre d'exemple, et faite en se référant aux dessins annexés, sur lesquels :
- la Figure 1 est une vue schématique en perspective éclatée d'un premier ensemble de culture selon l'invention, avec une nacelle occupant une position de manipulation ;
- la Figure 2 est une vue de côté de la nacelle dans l'ensemble de la Figure 1 ;
- la Figure 3 est une vue de dessus du premier dispositif de culture selon l'invention, la nacelle occupant sa position de culture ;
- la Figure 4 est une vue en perspective de trois-quarts face d'une nacelle d'un deuxième ensemble de culture selon l'invention ;
- la Figure 5 est une vue analogue à la Figure 3 du deuxième ensemble de culture selon l'invention ;
- la Figure 6 est une vue de dessous du deuxième ensemble de culture selon l'invention ;
- la Figure 7 est une vue en perspective d'une nacelle d'un troisième ensemble de culture selon l'invention ;
- la Figure 8 est une vue de côté de la nacelle de la Figure 7 ;
- la Figure 9 est une vue de dessus du support de nacelle du troisième ensemble de culture selon l'invention ;
- la Figure 10 est une vue prise en coupe suivant le plan vertical X-X de la Figure 9.

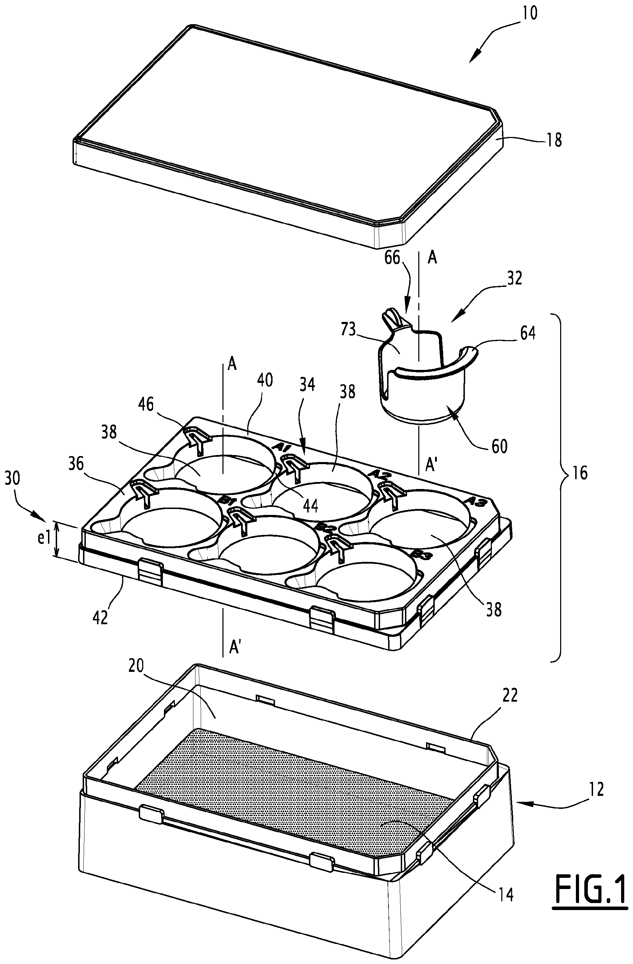

Un premier ensemble de culture 10 selon l'invention est représenté sur les Figures 1 à 3.

Cet ensemble 10 est destiné à cultiver in vitro différents types de cellules et/ou de tissus ou à maintenir ex-vivo différents tissus, en vue de leur utilisation pour différents tests ou évaluations.

Le premier ensemble 10 comprend, de bas en haut sur la Figure 1, un bac 12 de réception d'un milieu de culture 14, un premier dispositif 16 de culture selon l'invention, destiné à être monté sur le bac 12, et un couvercle 18 destiné à coiffer le bac 12 et le dispositif 16, de manière à assurer la stérilité du système. Les échanges gazeux entre l'intérieur du bac 12 recouvert de son couvercle 18 sont autorisés par l'intermédiaire de jours. Toutefois, les jours permettant ces échanges sont de taille telle qu'ils empêchent les contaminations bactériennes.

Le bac 12 délimite au moins une cavité 20 de réception du milieu 14 et une surface supérieure 22 de montage du dispositif 16, s'étendant le long du bord supérieur du bac 12.

La cavité 20 débouche vers le haut à travers la surface supérieure 22. Dans l'exemple représenté sur la Figure 1, le bac 12 présente une cavité 20 unique contenant un milieu de culture 14 commun.

En variante, le bac 12 délimite une pluralité de cavités 20, désignées par le terme « puits », séparées de manière étanche les unes des autres pour contenir différents milieux.

Le milieu 14 est avantageusement un liquide. Il est propre à imprégner les cellules ou tissus à cultiver pour favoriser leur croissance et les maintenir en vie. Le liquide est par exemple une solution aqueuse de différents nutriments tels que des sels minéraux, des réactifs, des vitamines ... Le milieu 14 peut être solide. Dans ce cas, il est dit « gélosé ».

Le dispositif de culture 16 comprend un support de nacelle 30 destiné à être engagé au dessus du bac 12, au moins une nacelle 32 de culture destinée à être portée par le support de nacelle 30 et, selon l'invention, des moyens 34 de retenue libérables de chaque nacelle 32 sur le support 30, qui seront décrits en détail plus bas.

Le support 30 comporte un corps 36 sensiblement plat délimitant une pluralité d'ouvertures 38 de réception de nacelles.

Dans l'exemple représenté sur la Figure 1, le corps 36 délimite six ouvertures de réception 38. Plus généralement, le nombre d'ouvertures de réception 38 est avantageusement égal au nombre de nacelles 32 et est compris entre 1 et 96.

Le corps 36 s'étend entre une surface supérieure 40 de retenue des nacelles 32 et une surface inférieure 42 de montage sur le bac 12.

Le corps 36 présente avantageusement une épaisseur, prise entre les surfaces 40 et 42 inférieure à ses autres dimensions.

Le corps 36 présente un contour de forme sensiblement complémentaire au contour extérieur du bac 12 le long de son bord supérieur. Dans l'exemple représenté sur la Figure 1, le contour est rectangulaire.

La surface supérieure 40 est sensiblement horizontale lorsque le corps 36 est monté sur le bac 12. La surface supérieure 40 délimite, à la périphérie de chaque ouverture de réception 38, un premier gradin 44 de retenue de nacelle, et un deuxième gradin 46 de retenue de nacelle situé à l'opposé du premier gradin 44.

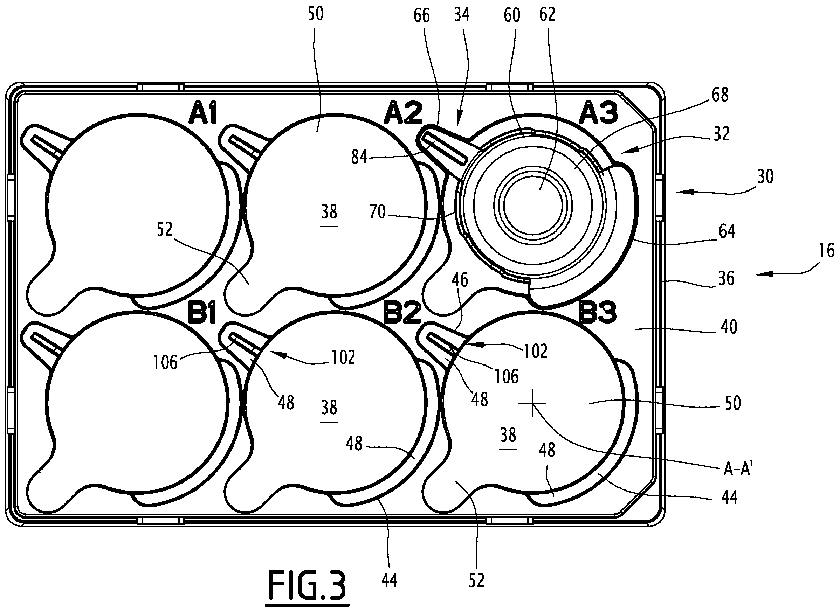

En référence à la Figure 3, chaque gradin 44, 46 délimite une surface de fond 48 sensiblement horizontale, située légèrement au dessous du niveau moyen de la surface supérieure 40.

Chaque ouverture de réception 38 s'étend le long d'un axe vertical A-A' d'insertion de la nacelle dans l'ouverture 38, entre la surface inférieure 42 et la surface supérieure 40. Chaque ouverture de réception 38 est traversante, de sorte qu'elle débouche dans les surfaces 40 et 42.

Chaque ouverture 38 présente une section horizontale sensiblement constante sur toute sa hauteur à travers le corps 36.

Comme illustré par la Figure 3, chaque ouverture 38 comprend une région centrale 50 de section extérieure sensiblement analogue à la section extérieure d'une nacelle 32 et une échancrure périphérique 52 qui prolonge vers l'extérieur la région centrale 50 radialement par rapport à l'axe A-A'.

Dans cet exemple, la région centrale 50 est de contour circulaire.

L'échancrure périphérique 52 débouche dans la région centrale 50. Elle s'étend angulairement autour de l'axe A-A' dans un secteur angulaire d'étendue inférieure à 30°.

L'échancrure 52 délimite ainsi, lorsque la nacelle 32 est insérée dans la région 50, un passage d'accès au milieu de culture contenu dans la cavité 20. Un outil de prélèvement, de remplissage ou de dosage, tel qu'une pipette, peut donc être introduit dans l'échancrure 52 pour alimenter en milieu de culture ou prélever ou ajouter toute autre substance dans le milieu de culture, sans avoir à déplacer la nacelle 32.

Dans l'exemple représenté sur les Figures 1 à 3, le support 30 est mobile par rapport au bac de base 12 entre une position engagée sur le bac 12, dans laquelle le support 14 obture sensiblement la ou chaque cavité 20, à l'exception des ouvertures de passage 38, et une position démontée à l'écart du bac 12 pour la manipulation conjointe des nacelles 32 retenues dans le support 30, comme on va le décrire en détail plus bas.

Lorsque le support 30 occupe sa position montée sur le bac 12, la surface supérieure de retenue 40 est orientée vers le haut.

Dans l'exemple représenté sur les Figures 1 à 3, chaque nacelle 32 comprend un récipient 60 creux, un substrat de culture 62 rapporté dans le récipient 60, un rebord d'appui 64 sur la surface de retenue 40, en forme de collerette, et un bras 66 d'appui et de manipulation de la nacelle 32.

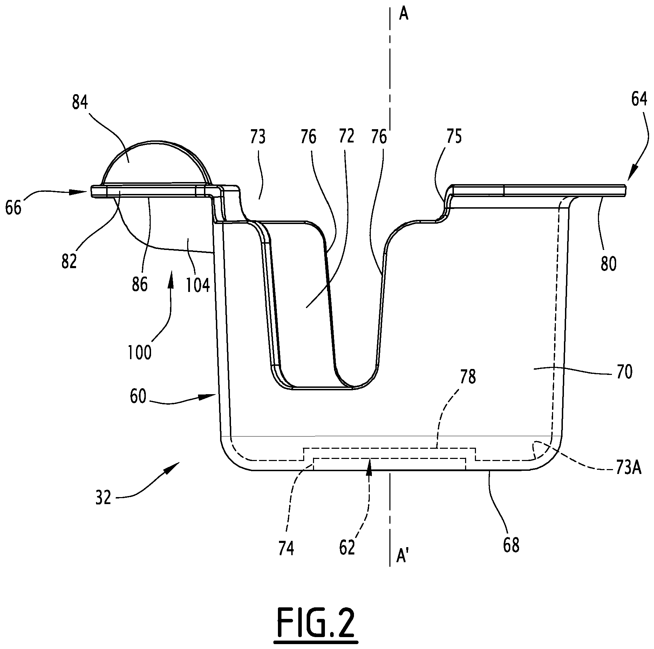

Le récipient 60 comprend une paroi de fond 68 sensiblement horizontale sur la Figure 2 et une paroi périphérique latérale 70 qui délimitent un volume intérieur 72 de culture débouchant par une ouverture supérieure 73. La jonction 73A entre la paroi de fond 68 et la paroi périphérique latérale 70 est avantageusement arrondie dans le volume intérieur 72, afin d'assurer un écoulement idéal des différents liquides qui sont susceptibles d'être déposés dans le volume intérieur 72.

La paroi de fond 68 s'étend sensiblement perpendiculairement à l'axe d'insertion A-A' de la nacelle 32 dans une ouverture de réception.

La paroi de fond 68 délimite un passage central 74 d'alimentation en milieu de culture débouchant dans le volume intérieur 72. Dans l'exemple représenté sur les Figures 1 à 3, le passage central 74 est d'axe A-A' et de forme sensiblement circulaire.

Il présente une surface variable en fonction du substrat de culture et généralement supérieure à 10% de la surface délimitée par la paroi de fond.

La paroi latérale 70 fait saillie vers le haut à partir de la paroi de fond 68 jusqu'à un bord supérieur 75. Elle présente une forme sensiblement cylindrique d'axe A-A'. Elle délimite deux encoches latérales 76 d'accès au volume intérieur 72 situés de part et d'autre d'un plan vertical médian passant par l'axe A-A'.

Les encoches 76 débouchent vers le haut autour de l'ouverture supérieure 73. La hauteur des encoches 76, prise parallèlement à l'axe A-A' entre le bord supérieur 75 et la paroi de fond 68 est supérieure à l'épaisseur du corps 36, prise suivant l'axe A-A' afin de garantir d'importants échanges gazeux entre les différents compartiments du dispositif.

Ainsi, les encoches 76 autorisent un accès aisé au volume intérieur 72, notamment pour le prélèvement, à l'aide d'un outil de prise tel qu'une pince, de l'échantillon cultivé dans le volume 72.

Dans une variante non représentée, la paroi latérale porte des entretoises faisant saillie extérieurement à partir de la paroi pour empêcher le contact entre la paroi 70 et le corps 36 dans l'ouverture de réception 38 et limiter les remontées de milieu de culture par capillarité.

Le substrat de culture 62 est rapporté sur la paroi de fond 68 en partie supérieure ou inférieure autour du passage 74. Ce substrat obture le passage 74. Le substrat 62 est perméable pour permettre l'entrée de milieu de culture dans le volume intérieur 72 à travers le passage 74. Il définit une surface supérieure de culture 78 destinée à recevoir les cellules ou tissus à cultiver. Il est réalisé par exemple à base d'une éponge de collagène, ou d'un film de collagène ou à base d'une matrice en polycarbonate, ou d'un tissu synthétique tel que le nylon ou encore d'une grille métallique.

Dans une variante (non représentée), le substrat 62 est formé directement dans la paroi de fond 68 en étant venu de matière avec la paroi de fond. Il délimite une pluralité d'ouvertures de passages 74 pour la diffusion de milieux de culture dans le volume 72.

Dans une autre variante, le substrat 62 est imperméable et au moins un passage d'alimentation 74 est ménagé à l'écart de la surface de culture 78 dans la paroi de fond 68 ou dans une partie inférieure de la paroi latérale 70 située au voisinage de la paroi de fond 68.

Dans l'exemple représenté sur les Figures 1 à 3, le rebord d'appui 64 est venu de matière avec le récipient 60. Il s'étend radialement à l'écart de l'axe A-A' perpendiculairement à cet axe A-A'. Il présente une section horizontale, prise perpendiculairement à l'axe A-A', analogue à la section horizontale définie par le premier gradin 44.

Le rebord 64 définit une première surface inférieure d'appui 80 destinée à être appliquée sur la surface de retenue 40 dans la position de culture.

Le bras 66 s'étend radialement à l'écart de l'axe A-A' à l'opposé du rebord 64. Il comprend un plateau d'appui 82 sensiblement horizontal et un ergot de saisie 84 qui fait saillie vers le haut à partir du plateau 82.

Le plateau 82 définit une deuxième surface inférieure d'appui 86 sur la surface de retenue 40, de section horizontale sensiblement analogue à la section horizontale du deuxième gradin 46.

Le bras 66 présente une étendue angulaire, prise autour de l'axe A-A', inférieure à l'étendue angulaire du rebord 64.

La hauteur utile de la nacelle 32, définie entre les première et deuxième surfaces d'appui 80, 86 et la paroi de fond 68, est supérieure à l'épaisseur e1 du corps 36.

Chaque nacelle 32 est mobile par rapport au support 30 entre une position de culture, représentée sur la Figure 3, dans laquelle elle est engagée dans une ouverture de réception 38, et une position de manipulation, représentée sur la Figure 1, dans laquelle la nacelle 32 est disposée à l'écart du support 30 pour être déplaçable librement par rapport au support 30.

Dans la position de culture, la nacelle 32 a été insérée dans une ouverture de réception 38, parallèlement à l'axe A-A'.

La première surface d'appui 80 est appliquée contre la surface du premier gradin 44 sur la surface de retenue 40.

La deuxième surface d'appui 86 est appliquée contre la surface du deuxième gradin 46 sur la surface supérieure de retenue 40.

La paroi de fond 68 fait saillie vers le bas au delà de la surface inférieure 42 pour être apte à tremper dans le milieu de culture 14 lorsque le support 30 est monté sur le bac 12.

L'ergot 84 de saisie fait saillie au-dessus de la surface supérieure de retenue 40 pour être saisi facilement par un utilisateur du dispositif à l'aide de ses doigts ou à l'aide d'un outil de saisie tel qu'une pince.

Selon l'invention, et dans le mode de réalisation des Figures 1 à 3, les moyens de retenue libérables 34 comprennent un organe de retenue formé par une saillie de retenue 100 solidaire de la nacelle 32, et un organe de retenue complémentaire 102 d'enserrement de la saillie de retenue 100, solidaire du corps 36 au voisinage de chaque ouverture 38.

Dans l'exemple représenté sur la Figure 2, la saillie 100 est venue de matière avec la nacelle 32. Elle est formée par une lame verticale 104 s'étendant radialement par rapport à l'axe A-A' sous le plateau 82 et à l'extérieur de la paroi latérale 70 au voisinage du bord supérieur 75. La lame 104 présente une épaisseur sensiblement constante.

Un organe d'enserrement 102 est formé dans le corps 36 au voisinage de chaque ouverture de réception 38.

Chaque organe 102 définit une gorge 106 de réception de la saillie, de largeur légèrement inférieure à celle de la lame 104. La gorge 106 s'étend radialement vers l'extérieur à partir de l'ouverture de réception 38 par rapport à l'axe A-A'. Elle débouche vers le haut dans la paroi de fond 48 du deuxième gradin 46.

Ainsi, dans la position de culture, la saillie 100 est insérée en force dans la gorge 106 définie par l'organe d'enserrement 102. Dans cette position, les moyens de retenue 34 coopèrent pour retenir la nacelle 32 dans l'ouverture 38 lors d'un retournement conjoint du support 30 et la nacelle 32, dans lequel la surface d'appui 40 s'oriente vers le bas et dans lequel les surfaces d'appui 80 et 86 s'orientent vers le haut.

Ainsi, lorsque les moyens de retenue libérables 34 coopèrent, le support 30 et la nacelle 32 sont déplaçables conjointement quelque soit l'orientation de la surface de retenue 40.

Toutefois, il est possible de libérer les moyens de retenue 34 de la nacelle 32 par rapport au support 30 en appliquant sur la nacelle 32 une force manuelle de traction à l'écart du corps 36 de manière réversible, sans arrachement ni détérioration irréversible du support 30 et du corps 36.

Pour ce faire, la saillie 100 est déplacée hors de la gorge 106, ce qui permet l'extraction de la nacelle 32 hors de l'ouverture de réception 38 sensiblement le long de l'axe A-A'.

Le couvercle 18 est propre à coiffer le dispositif 16 et le bac 12 pour délimiter, à l'intérieur de l'ensemble de culture 10, un espace de culture présentant une atmosphère contrôlée et stérile et contenant les nacelles 32.

Dans une position auxiliaire de culture, la nacelle 32 est juste posée sur la surface supérieure 40 du corps sans engagement des moyens de retenue 34. La nacelle 32 n'est alors pas maintenue dans l'ouverture de réception 38 lors d'un retournement conjoint du support 30 et de la ou de chaque nacelle 32 qui oriente la surface de retenue 40 vers le bas.

Le fonctionnement du premier ensemble 10 selon l'invention va maintenant être décrit.

Initialement, chaque nacelle 32 est chargée avec des cellules et/ou un tissu à cultiver.

A cet effet, les cellules et/ou le tissu sont déposés sur la surface de culture 78. Le milieu de culture 14 est par ailleurs introduit dans la cavité 20.

Chaque nacelle 32 est montée dans sa position de culture sur le support 30. A cet effet, chaque récipient creux 60 est introduit dans une ouverture de réception 38 depuis la surface supérieure 40, jusqu'à ce que la première surface d'appui 80 et la deuxième surface d'appui 86 soient appliquées sur les surfaces de fond 48 définies respectivement sur le premier gradin 44 et sur le deuxième gradin 46 de la surface supérieure de retenue 40.

Lors de ce déplacement, la saillie 100 formée par la lame 104 pénètre en force dans la gorge 106 délimitée par l'organe d'enserrement 102, ce qui immobilise la nacelle 32 par rapport au support 30 dans la position de culture.

Puis, le dispositif de culture 16 est monté sur le bac 12. Lors du passage du dispositif 16 de la position démontée à la position montée, la paroi de fond 68 de chaque nacelle 32 s'immerge dans le milieu de culture 14 pour imbiber les cellules et/ou le tissu à travers le passage d'alimentation 74 et le substrat perméable 62.

Le couvercle 18 est alors refermé pour coiffer le dispositif 16.

En variante, le support 30 est monté sur le bac 12 avant la disposition des nacelles 32 dans les ouvertures de réception 38.

Lorsqu'un prélèvement et/ou un test doit être effectué individuellement sur une nacelle donnée 32, un opérateur saisit l'ergot 84 et extrait la saillie 100 hors de la gorge 106. Il déplace alors la nacelle 32 à l'écart de l'ouverture de réception 38 pour effectuer le prélèvement et/ou le test, tout en maintenant éventuellement le support 30 monté sur le bac 12.

Au contraire, lorsque plusieurs nacelles 32 d'un groupe de nacelles chargées sur le support 30 doivent être manipulées ensemble, le support 30 et les nacelles 32 dans leurs positions de culture sont déplacés conjointement à l'écart du bac 12. La présence des moyens de retenue libérables 34 permet éventuellement de retourner le support 30 pour placer la surface supérieure de retenue 40 orientée vers le bas, tout en maintenant les nacelles 32 solidaires du support 30.

En outre, lorsque les nacelles 32 sont retenues dans le support 30, l'ensemble des parois de fond 68 et des substrats 62 sont avantageusement tous maintenus dans le même plan. La disposition des nacelles 32 dans le corps 36 et la disposition du corps 36 relativement au bac 12 permet avantageusement de placer les parois de fond 68 et les substrats 62 dans un même plan horizontal, parallèle au plan sur lequel le bac est disposé.

Avantageusement, il est alors possible de disposer chaque substrat 62 à une même position dite d'interface air-liquide ou encore d'effectuer un traitement thermique simultané, par exemple de congélation par contact.

De plus, lorsque les échantillons contenus dans les nacelles 32 doivent être agités, il est possible d'agiter de manière robuste le support 30, sans risque de détachement des nacelles 32 puisque les moyens de retenue 34 maintiennent les nacelles 32 dans leurs positions de culture.

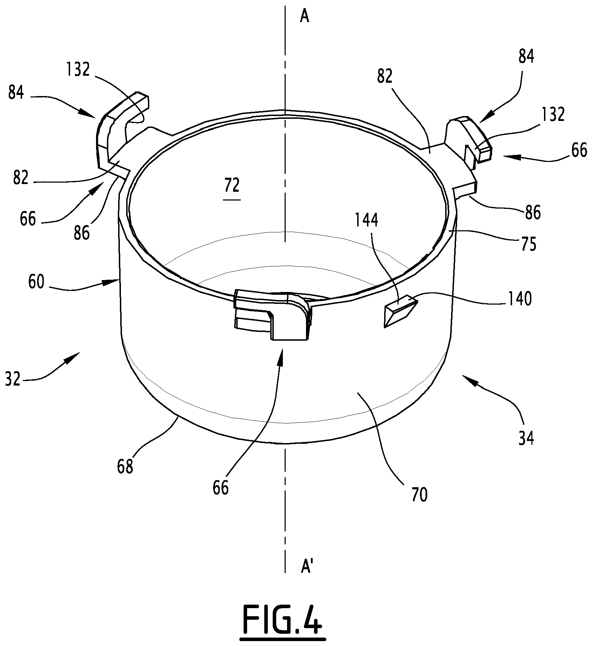

Un deuxième ensemble 130 selon l'invention est représenté partiellement sur les Figures 4 à 6.

A la différence du premier ensemble 10, les nacelles 32 du deuxième ensemble, telles qu'illustrées sur la Figure 1, comprennent trois bras d'appui 66 répartis à la périphérie de la paroi latérale 70. La paroi latérale 70 est par ailleurs dépourvue d'encoches 76 et présente une hauteur sensiblement constante autour de l'axe A-A'.

Chaque ergot de saisie 84 est formé par un crochet 132 qui fait saillie verticalement le long du bord extérieur du plateau 82.

La surface supérieure de retenue 40 délimite trois gradins 134 répartis autour de la périphérie de chaque ouverture de réception 38. Chaque gradin 134 présente une étendue angulaire, prise autour de l'axe A-A', supérieure à l'étendue angulaire autour de l'axe A-A' de chaque bras 66.

En outre, chaque gradin 134 définit deux bords radiaux 136 de blocage en rotation de la nacelle 32, comme on va le décrire plus bas.

Dans le deuxième ensemble 130 selon l'invention, les moyens de retenue libérable 34 de la nacelle diffèrent des moyens de retenue 34 du premier ensemble 10 en ce que les organes de retenue et les organes de retenue complémentaires sont formés respectivement par des butées 140 solidaires de la nacelle et par des butées complémentaires 142 solidaires du corps 30, formant un mécanisme de type « baïonnette ».

Comme illustré par la Figure 4, chaque butée 140 fait saillie radialement vers l'extérieur par rapport à la paroi latérale 70 de la nacelle 32. Chaque butée 140 définit une surface supérieure de butée 144 sensiblement horizontale.

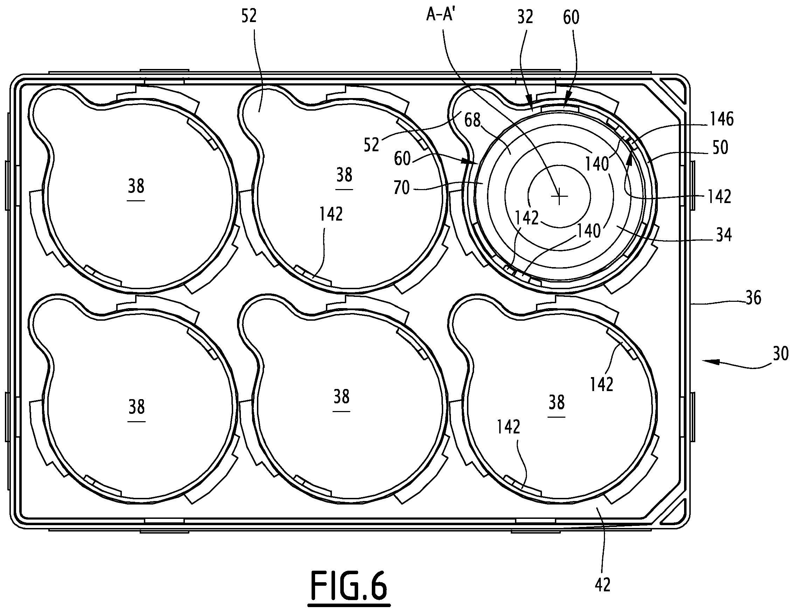

En référence à la Figure 6, chaque butée complémentaire 142 fait saillie radialement vers l'axe A-A' dans l'ouverture de réception 38 à partir du corps 36. Chaque butée complémentaire 142 définit une surface inférieure 146 sensiblement horizontale.

Lorsque les surfaces d'appui 86 sont disposées en appui sur la surface supérieure de retenue 40, la nacelle 32 est montée mobile en rotation autour de l'axe A-A' dans l'ouverture de réception 38 entre une position d'insertion de la nacelle 32 dans l'ouverture de réception 38 et la position de culture.

Dans la position d'insertion, la butée 140 et la butée complémentaire 142 sont situées angulairement à l'écart l'une de l'autre autour de l'axe A-A'.

Ainsi, dans cette position, l'espace situé au-dessus de la butée 140 parallèlement à l'axe A-A' est dégagé et la nacelle 32 peut être extraite librement hors de l'ouverture de réception 38.

Dans la position verrouillée de culture, la nacelle 32 a été pivotée autour de l'axe A-A' pour amener la surface inférieure de butée 146 en regard axialement et angulairement de la surface supérieure de butée 144. La butée complémentaire 142 est située juste au-dessous de la butée 140 et obture l'espace situé au dessus de cette butée 140 parallèlement à l'axe A-A'.

Les deux butées 142, 144 sont alors en contact pour empêcher l'extraction vers le haut de la nacelle 32 hors de l'ouverture de réception 38.

Dans cette position, chaque plateau d'appui 82 coopère avec un bord de blocage 136 d'un gradin pour empêcher le déplacement en rotation de la nacelle autour de l'axe A-A' dans un premier sens au-delà de la position verrouillée de culture.

Lors de l'utilisation du deuxième ensemble 130, chaque nacelle 32 est introduite dans une ouverture de retenue 38 en la déplaçant le long de l'axe A-A' pour l'amener dans la position d'insertion. Lors de ce déplacement, chaque butée 140 passe successivement au dessus, latéralement en regard, puis au dessous des butées complémentaires 142.

Puis, la nacelle 32 est pivotée autour de l'axe A-A' en faisant glisser chaque bras 66 sur un gradin 134 dans le premier sens jusqu'à ce que le plateau 82 bute latéralement contre un bord de blocage 136.

Lors de ce déplacement, chaque butée 140 passe angulairement en regard d'une butée complémentaire 142. La coopération entre la surface supérieure 144 de la butée 140 et la surface inférieure 146 de la butée complémentaire 142 bloque alors la nacelle 32 dans sa position de culture.

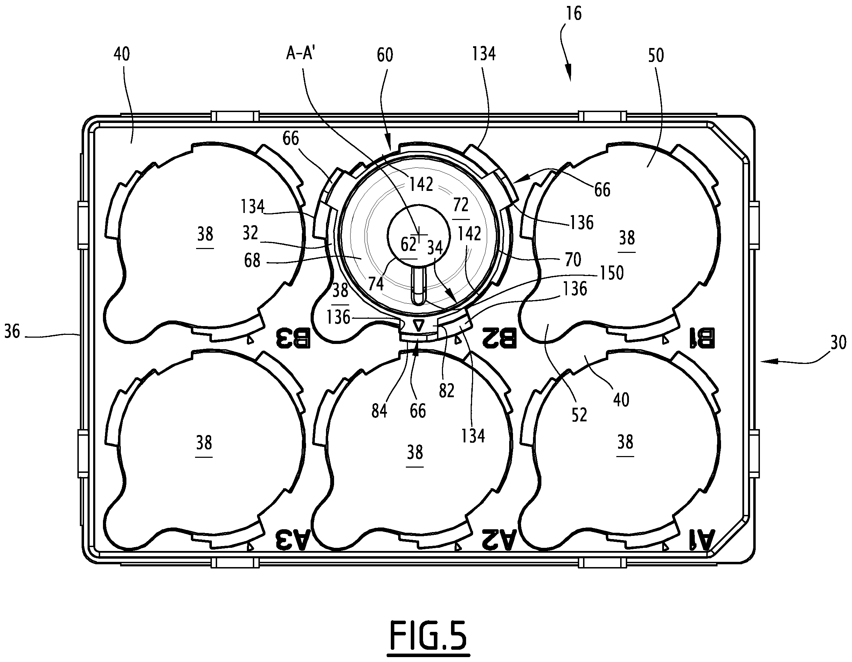

Dans une variante représentée sur la Figure 5, et applicable au mode de réalisation des Figures 1 à 3, la paroi de fond 68 délimite une gorge radiale 150 de guidage de liquide débouchant dans la surface de culture 78. Ainsi, un opérateur peut introduire l'extrémité inférieure d'un outil de dosage de liquide dans le volume 72 à travers l'ouverture supérieure 73 et déposer du liquide dans la gorge 150 pour l'amener à la surface de culture 78, sans risque d'endommager la culture présente sur la surface 78.

Dans une variante (non représentée), les moyens de retenue libérables 34 comprennent des moyens de vissage de la nacelle 32 dans le corps 36.

Ces moyens de vissage comprennent par exemple un filet extérieur solidaire de la nacelle 32 et un filetage intérieur solidaire du corps 36 disposé dans l'ouverture 38.

Dans une autre variante (non représentée), les moyens de retenue libérables 34 sont formés par des moyens d'encliquetage.

Avantageusement, les ensembles selon l'invention sont réalisés au format SBS pour permettre une manipulation par un robot.

On notera que dans la position d'insertion définie plus haut, il est possible d'immerger partiellement chaque nacelle 32 dans le milieu de culture pour effectuer une culture cellulaire. Il n'est donc pas nécessaire que la nacelle 32 soit dans la position verrouillée pour effectuer une culture.

L'utilisation de moyens de retenue libérables 34 comprenant un mécanisme à baïonnette présente l'avantage d'éviter l'usure mécanique dans le temps des bras.

En outre, les surfaces inférieures 86 des bras 66 sont appliquées de manière ferme sur les surfaces de retenue 40, ce qui garantit une planéité parfaite de la paroi de fond 68 par rapport au bac de culture 12.

Ainsi, l'immersion de la paroi de fond 68 dans le milieu de culture 14 est identique en tous points du fond de la nacelle 32, ce qui garantit que le contenu de chaque nacelle 32 est placé précisément à l'interface entre l'air et le milieu de culture liquide.

La présence des moyens de retenue 34 de type baïonnette, et notamment la coopération entre la butée complémentaire 142 solidaire du support 30 et la butée 140 présente sur la nacelle 32, assure l'immobilité axiale de chaque nacelle 32 le long de l'axe A-A' d'insertion par rapport au support 30 lorsque la nacelle occupe la position verrouillée de culture.

Cette immobilisation de la nacelle 32 par rapport au support 30 est garantie quel que soit le sens de sollicitation de la nacelle le long de l'axe A-A'.

Ainsi, chaque nacelle 32 reste immobile lorsqu'une force est appliquée de bas en haut sur la paroi de fond 68, par exemple lorsque les nacelles 32 sont placées dans un même plan pour y subir un choc thermique. Lorsqu'une telle force est appliquée, chaque butée 140 présente sur la nacelle 32 est retenue par la butée complémentaire 142 présente sur le support 30.

De même, la coopération entre la surface inférieure 86 des bras 66 et la surface de retenue 40 garantit que la nacelle 32 reste immobile lorsqu'une force de poussée dirigée de haut en bas est appliquée sur la nacelle 32.

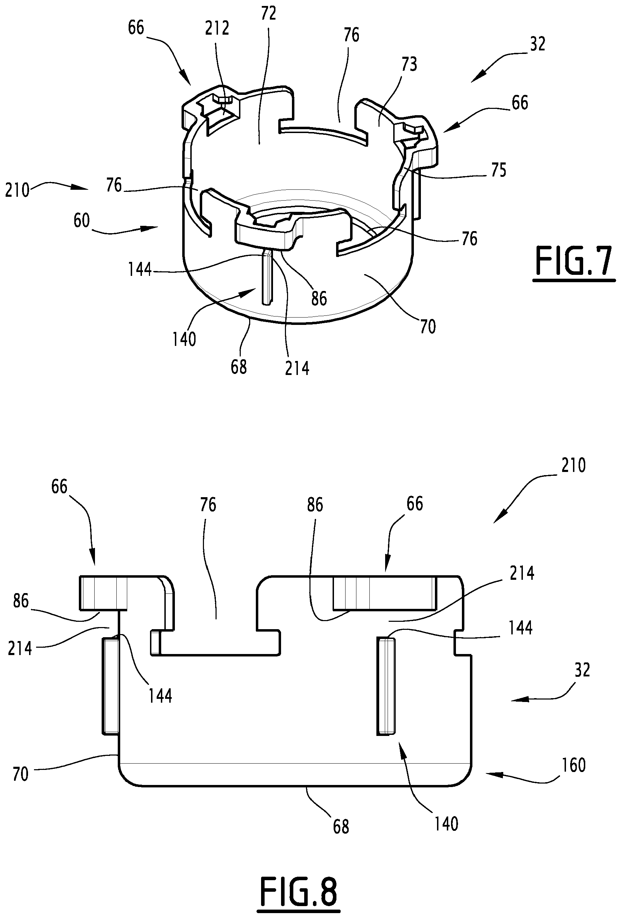

Un troisième ensemble de culture 210 selon l'invention est représenté partiellement sur les Figures 7 à 10.

A la différence du deuxième ensemble 130, les bras 66 de chaque nacelle 32 délimitent avantageusement une cavité creuse 212 de saisie propre à recevoir un outil destiné à manoeuvrer la nacelle entre sa position d'insertion et sa position verrouillée. Cet outil est par exemple une pince à deux ou trois branches.

Les cavités 212 débouchent vers le haut, et vers le bas parallèlement à l'axe A-A'.

Comme le deuxième ensemble 130, le troisième ensemble 210 présente des moyens de retenue 34 de type baïonnette.

Dans le troisième ensemble 310, chaque surface supérieure de butée 144 d'une butée 140 débouche en regard d'un bras 66, à l'opposé de la surface inférieure 86.

La surface inférieure 86 délimitée par chaque bras délimite ainsi avec la surface supérieure 144 de la butée 140 en regard, un passage 214 d'insertion de la butée complémentaire 142.

La butée 140 est formée dans cet exemple par une nervure axiale faisant saillie sur la paroi latérale 70.

La nacelle 32 du troisième ensemble 210 comprend avantageusement une encoche 76 dans la paroi latérale 70, ménagée entre chaque paire de bras 66 adjacents.

La hauteur des encoches 76 est inférieure à leur largeur maximale. Cette hauteur est avantageusement inférieure à 50% de la hauteur de la paroi latérale 70. Ce choix dimensionnel garantit les échanges entre l'intérieur de la nacelle 32 et l'extérieur, quelle que soit la position de la nacelle 32 entre sa position d'insertion et sa position verrouillée de culture.

Dans cet exemple, les encoches 76 sont chacune en forme générale de T inversé.

Comme dans le deuxième ensemble 130, le support 30 du troisième ensemble 210 comporte, autour de chaque ouverture 38, une pluralité de gradins 134 délimitant chacun une surface supérieure 40 de retenue d'un bras 66.

Les butées complémentaires 142 font saillie radialement dans l'ouverture de réception 38 vers l'axe A-A' de l'ouverture de réception 38.

Chaque butée 142 complémentaire présente une surface supérieure horizontale 216 qui affleure et prolonge radialement vers l'axe A-A' la surface supérieure de retenue 40, et une surface inférieure 146 destinée à coopérer avec la butée 140 portée par la nacelle 32.

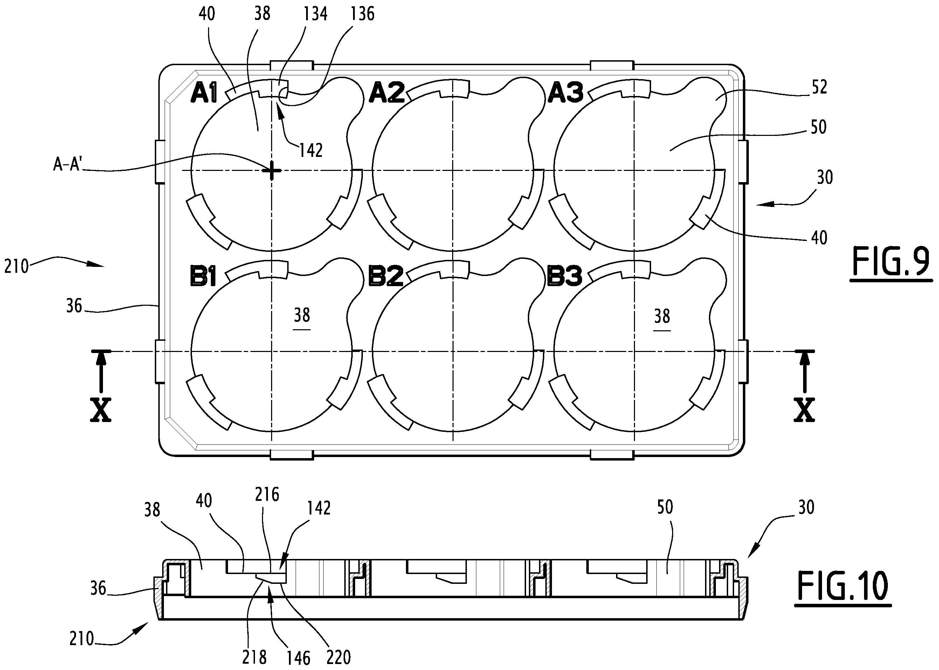

Dans l'exemple représenté sur la Figure 10, la surface inférieure 146 comprend une première partie 218 inclinée par rapport à l'horizontale, et une deuxième partie 220 sensiblement horizontale destinée à la retenue de la nacelle 32 dans sa position verrouillée de culture. La deuxième partie 220 est décalée angulairement autour de l'axe A-A' par rapport à la première partie 218.

Le fonctionnement du troisième ensemble 210 va maintenant être décrit.

Comme pour le deuxième ensemble 130, chaque nacelle 32 est d'abord introduite dans une ouverture de retenue 38 en la déplaçant vers le bas le long de l'axe A-A' pour l'amener dans la position d'insertion.

A cet effet, les bras 66 sont introduits dans chaque gradin 134 à l'écart angulairement de la butée complémentaire 142.

Lors de ce déplacement, la butée 140 passe successivement au-dessus, latéralement à l'écart puis au dessous des butées complémentaires 142.

Chaque butée complémentaire 142 est alors située en regard du passage d'insertion 214 délimité entre la surface inférieure 86 de chaque bras 66 et la surface supérieure 144 de la butée 140.

Puis, la nacelle 32 est pivotée autour de l'axe A-A' en faisant glisser chaque bras 66 sur un gradin 134, jusqu'à ce que le bras 66 bute contre un bord de blocage 136.

Lors de ce passage, la surface supérieure 144 de la butée 140 entre successivement en contact avec la première partie inclinée 218 de la butée complémentaire 142, puis s'introduit entre la surface inférieure 86 et la surface supérieure 144 dans le passage d'insertion 214, sous la deuxième partie 220.

La butée complémentaire 142 est alors enserrée entre les surfaces 86, 144 pour bloquer axialement le long de l'axe A-A' la nacelle 32 par rapport au support dans les deux sens le long de l'axe A-A'.

L'enserrement de la butée complémentaire est cependant ajusté pour tolérer un verrouillage et un déverrouillage manuel.

## Revendications

1. Dispositif (16) de culture cellulaire ou tissulaire, du type comprenant :
- un support (30), destiné à obturer au moins partiellement un bac (12) contenant un milieu de culture (14), le support (30) comprenant un corps (36) délimitant au moins une ouverture (38) traversante de réception de nacelle et au moins une surface (40) de retenue de nacelle destinée à être orientée vers le haut lorsque le support (30) obture le bac (12) ;
- au moins une nacelle (32) montée mobile par rapport au support (30) entre une position de culture dans laquelle la nacelle (32) est engagée à travers l'ouverture de réception (38) et une position de manipulation, dans laquelle la nacelle (32) est disposée à l'écart du support (30),
la nacelle (32) comprenant une paroi de fond (68) portant une surface de culture (78) et une paroi latérale (70) délimitant une ouverture supérieure (73), la nacelle (32) délimitant au moins un passage (74) d'alimentation de la surface de culture (78) en milieu de culture, distinct de l'ouverture supérieure (73) ;
dans lequel le dispositif (16) comprend des moyens (34) de retenue libérables de la ou de chaque nacelle (32) dans sa position de culture, les moyens de retenue libérables (34) étant propres à maintenir la ou chaque nacelle (32) dans l'ouverture de réception (38) lors d'un retournement conjoint du support (30) et de là ou de chaque nacelle (32) qui oriente la surface de retenue (40) vers le bas, et dans lequel les moyens de retenue (34) forment un mécanisme de type baïonnette.

2. Dispositif (16) selon la revendication 1, **caractérisé en ce que** dans la position de culture, au moins une surface d'appui (80, 86) de la nacelle (32) est appliquée contre la surface de retenue (40), les moyens de retenue libérables (34) étant propres à maintenir la surface d'appui (80, 86) de la nacelle (32) appliquée contre la surface de retenue (40) lors du retournement conjoint du support (30) et de la ou de chaque nacelle (32) qui oriente la surface de retenue (34) vers le bas.

3. Dispositif (16) selon l'une quelconque des revendications précédentes, **caractérisé en ce que** les moyens de retenue libérables (34) comprennent un organe de retenue (100 ; 140) solidaire d'un premier du support de nacelle (30) et de la nacelle (32) et un organe de retenue complémentaire (102 ; 142) solidaire d'un deuxième du support de nacelle (30) et de la nacelle (32) pour coopérer avec l'organe de retenue (100 ; 140) au moins lors du retournement conjoint du support (30) et de la ou de chaque nacelle (32).

4. Dispositif (16) selon la revendication 3, **caractérisé en ce que** l'organe de retenue (100) comprend une saillie, l'organe de retenue complémentaire (102) délimitant au moins une gorge (106) d'enserrement de la saillie, de dimension adaptée pour maintenir la saillie dans la gorge (106) par enserrement.

5. Dispositif (16) selon la revendication 3, **caractérisé en ce que** l'organe de retenue comprend une butée (140), l'organe de retenue complémentaire comprenant une butée complémentaire (142), la nacelle (32) étant déplaçable dans l'ouverture de réception (38) entre une position insérée dans laquelle l'espace situé au dessus de la butée (140) le long d'un axe (A-A') d'insertion de la nacelle (32) dans l'ouverture de réception (38) est dégagé, et la position de culture, dans laquelle la butée complémentaire (142) est disposée dans l'espace situé au dessus de la butée (140) le long d'un axe (A-A') d'insertion de la nacelle (32) dans l'ouverture de réception (38).

6. Dispositif (16) selon la revendication 5, **caractérisé en ce que** la nacelle (32) est montée rotative autour de l'axe d'insertion (A-A') entre la position d'insertion et la position de culture, les moyens de retenue libérables (34) comprenant avantageusement une surface (136) de blocage en rotation de la nacelle (32) dans sa position verrouillée de culture.

7. Dispositif (16) selon l'une quelconque des revendications 3 à 6, **caractérisé en ce que** la nacelle (32) comprend au moins un bras (66) faisant saillie transversalement vers l'extérieur par rapport à la paroi latérale (70), le bras (66) portant l'un de l'organe de retenue (100) et de l'organe de retenue complémentaire (102).

8. Dispositif selon l'une quelconque des revendications précédentes, **caractérisé en ce que** dans la position de culture, la nacelle (32) est immobile axialement le long de l'axe d'insertion (A-A').

9. Dispositif (16) selon l'une quelconque des revendications précédentes, **caractérisé en ce que** la paroi latérale (70) délimite au moins une encoche (76) débouchant vers le haut, l'encoche (76) s'étendant axialement sur une hauteur supérieure à l'épaisseur du corps (34).

10. Dispositif (16) selon l'une quelconque des revendications précédentes, **caractérisé en ce que** la paroi de fond (68) délimite une gorge (150) de guidage d'un liquide s'étendant radialement vers la surface de culture (72).

11. Dispositif (16) selon l'une quelconque des revendications précédentes, **caractérisé en ce que** la nacelle (32) comprend un organe (84) de saisie faisant saillie à l'écart et au dessus du support (30) lorsque la nacelle (32) occupe sa position de culture.

12. Dispositif (16) selon l'une quelconque des revendications précédentes, **caractérisé en ce que** la ou chaque ouverture de réception (38) comprend une région centrale (50) propre à recevoir la nacelle (32) dans la position de culture et une échancrure périphérique (52) s'étendant radialement à l'écart de la région centrale (50) pour permettre l'insertion d'un outil dans le bac (12) à travers le support (30) lorsque la nacelle (32) est reçue dans la région centrale (50).

13. Ensemble (10 ; 130) de culture cellulaire ou tissulaire, **caractérisé en ce qu'**il comprend :
- un bac (12) présentant au moins une cavité (20) destinée à recevoir un milieu de culture (14) ;
- un dispositif (16) selon l'une quelconque des revendications précédentes, le support (30) étant monté sur le bac (12) pour obturer partiellement la cavité (20).

14. Ensemble (10 ; 130) selon la revendication 13, **caractérisé en ce que** le support (30) est monté sur le bac (12) de manière à ce qu'une surface de culture d'une paroi de fond (68) d'une nacelle (32) soit disposée horizontalement.

15. Ensemble (10 ; 130) selon l'une des revendications 13 ou 14, **caractérisé en ce qu'**il comprend un couvercle (18) propre à coiffer le support (30), le support (30) et la ou chaque nacelle (32) étant reçus de manière stérile entre le bac (12) et le couvercle (18), dans un même plan.

## Patentansprüche

1. Zell- oder Gewebekultur-Vorrichtung (16) des Typs, der Folgendes umfasst:
- einen Träger (30), der dazu bestimmt ist, einen Behälter (12), der ein Kulturmedium (14) enthält, wenigstens teilweise zu verschließen, wobei der Träger (30) einen Körper (36), der wenigstens eine Durchgangsöffnung (38) für die Aufnahme eines Schälchens begrenzt, und wenigstens eine Oberfläche (40) zum Halten des Schälchens, die dazu bestimmt ist, nach oben orientiert zu werden, wenn der Träger (30) den Behälter (12) verschließt, umfasst;
- wenigstens ein Schälchen (32), das beweglich in Bezug auf den Träger (30) zwischen einer Kulturposition, in der das Schälchen (32) durch die Aufnahmeöffnung (38) hindurch in Eingriff ist, und einer Handhabungsposition, in der das Schälchen (32) in einem Abstand von dem Träger (30) angeordnet ist, montiert ist,
wobei das Schälchen (32) eine Bodenwand (68), die eine Kulturoberfläche (78) trägt, und eine Seitenwand (70), die eine obere Öffnung (73) begrenzt, umfasst, wobei das Schälchen (32) wenigstens einen Durchlass (74) für die Versorgung der Kulturoberfläche (78) mit dem Kulturmedium, der von der oberen Öffnung (73) verschieden ist, begrenzt;
wobei die Vorrichtung (16) Mittel (34) zum freigebbaren Halten des oder jedes Schälchens (32) in seiner Kulturposition umfasst, wobei die freigebbaren Haltemittel (34) das oder jedes Schälchen (32) in der Aufnahmeöffnung (38) halten können, wenn der Träger (30) und das oder jedes Schälchen (32) gemeinsam gedreht werden, wodurch die Halteoberfläche (40) nach unten orientiert ist, wobei die Haltemittel (34) einen Mechanismus des Bajonetttyps bilden.

2. Vorrichtung (16) nach Anspruch 1, **dadurch gekennzeichnet, dass** in der Kulturposition wenigstens eine Abstützoberfläche (80, 86) des Schälchens (32) gegen die Halteoberfläche (40) gedrückt wird, wobei die freigebbaren Haltemittel (34) die Abstützoberfläche (80, 86) des Schälchens (32) gegen die Halteoberfläche (40) gedrückt halten können, wenn der Träger (30) und das oder jedes Schälchen (32) gemeinsam gedreht werden, wodurch die Halteoberfläche (34) nach unten orientiert wird.

3. Vorrichtung (16) nach einem der vorhergehenden Ansprüche, **dadurch gekennzeichnet, dass** die freigebbaren Haltemittel (34) ein Halteorgan (100; 140), das mit einem Ersten des Schälchenträgers (30) und des Schälchens (32) fest verbunden ist, und ein komplementäres Halteorgan (102; 142), das mit einem Zweiten des Schälchenträgers (30) und des Schälchens (32) fest verbunden ist, um mit dem Halteorgan (100; 140) wenigstens bei der gemeinsamen Drehung des Trägers (30) und des oder jedes Schälchens (32) zusammenzuwirken, umfassen.

4. Vorrichtung (16) nach Anspruch 3, **dadurch gekennzeichnet, dass** das Halteorgan (100) einen Vorsprung aufweist, wobei das komplementäre Halteorgan (102) wenigstens eine Kehle (106) zum Einklemmen des Vorsprungs begrenzt, deren Abmessung dafür ausgelegt ist, dass der Vorsprung in der Kehle (106) durch Einklemmen gehalten wird.

5. Vorrichtung (16) nach Anspruch 3, **dadurch gekennzeichnet, dass** das Halteorgan einen Anschlag (140) umfasst, wobei das komplementäre Halteorgan einen komplementären Anschlag (142) umfasst, wobei das Schälchen (32) in der Aufnahmeöffnung (38) zwischen einer eingesetzten Position, in der der Raum, der sich über dem Anschlag (140) längs der Einsetzachse (A-A') des Schälchens (32) in die Aufnahmeöffnung (32) befindet, freigegeben ist, und der Kulturposition, in der der komplementäre Anschlag (142) in dem Raum angeordnet ist, der sich über dem Anschlag (140) längs einer Achse (A-A') des Einsetzens des Schälchens (32) in die Aufnahmeöffnung (38) befindet, verlagerbar ist.

6. Vorrichtung (16) nach Anspruch 5, **dadurch gekennzeichnet, dass** das Schälchen (32) drehbar um die Einsetzachse (A-A') zwischen der Einsetzposition und der Kulturposition montiert ist, wobei die freigebbaren Haltemittel (34) vorteilhaft eine Oberfläche (136) für die Blockierung der Drehung des Schälchens (32) in seiner verriegelten Kulturposition umfassen.

7. Vorrichtung (16) nach einem der Ansprüche 3 bis 6, **dadurch gekennzeichnet, dass** das Schälchen (32) wenigstens einen Arm (66) aufweist, der in Bezug auf die Seitenwand (70) transversal nach außen vorsteht, wobei der Arm (66) entweder das Halteorgan (100) oder das komplementäre Halteorgan (102) trägt.

8. Vorrichtung nach einem der vorhergehenden Ansprüche, **dadurch gekennzeichnet, dass** in der Kulturposition das Schälchen (32) axial längs der Einsetzachse (A-A') unbeweglich ist.

9. Vorrichtung (16) nach einem der vorhergehenden Ansprüche, **dadurch gekennzeichnet, dass** die Seitenwand (70) wenigstens eine Kerbe (76) begrenzt, die nach oben mündet, wobei sich die Kerbe (76) axial zu einer Höhe erstreckt, die höher als die Dicke des Körpers (34) ist.

10. Vorrichtung (16) nach einem der vorhergehenden Ansprüche, **dadurch gekennzeichnet, dass** die Bodenwand (68) eine Kehle (150) für die Führung einer Flüssigkeit begrenzt, die sich radial zu der Kulturoberfläche (72) erstreckt.

11. Vorrichtung (16) nach einem der vorhergehenden Ansprüche, **dadurch gekennzeichnet, dass** das Schälchen (32) ein Greiforgan (84) aufweist, das in einem Abstand von dem und über den Träger (30) vorsteht, wenn das Schälchen (32) seine Kulturposition einnimmt.

12. Vorrichtung (16) nach einem der vorhergehenden Ansprüche, **dadurch gekennzeichnet, dass** die oder jede Aufnahmeöffnung (38) einen mittleren Bereich (50) aufweist, der das Schälchen (32) in seiner Kulturposition aufnehmen kann, und einen Umfangseinschnitt (52) aufweist, der sich radial in einem Abstand von dem mittleren Bereich (50) erstreckt, um das Einführen eines Werkzeugs in den Behälter (12) durch den Träger (30) zu ermöglichen, wenn das Schälchen (32) in dem mittleren Bereich (50) aufgenommen ist.

13. Anordnung (10; 130) einer Zell- oder Gewebekultur, **dadurch gekennzeichnet, dass** sie Folgendes umfasst:
- einen Behälter (12), der wenigstens einen Hohlraum (20) aufweist, der dazu bestimmt ist, ein Kulturmedium (14) aufzunehmen;
- eine Vorrichtung (16) nach einem der vorhergehenden Ansprüche, wobei der Träger (30) an dem Behälter (12) montiert ist, um den Hohlraum (20) teilweise zu verschließen.

14. Anordnung (10; 130) nach Anspruch 13, **dadurch gekennzeichnet, dass** der Träger (30) an dem Behälter (12) in der Weise montiert ist, dass eine Kulturoberfläche einer Bodenwand (68) eines Schälchens (32) horizontal angeordnet ist.

15. Anordnung (10; 130) nach einem der Ansprüche 13 oder 14, **dadurch gekennzeichnet, dass** sie einen Deckel (18) umfasst, der den Träger (30) bedecken kann, wobei der Träger (30) und das oder jedes Schälchen (32) zwischen dem Behälter (12) und dem Deckel (18) in derselben Ebene steril aufgenommen sind.

## Claims

1. Cell or tissue culture device (16), of the type comprising:
- a carrier (30) for at least partially closing a tray (12) containing a culture medium (14), the carrier (30) comprising a body (36) which delimits at least one insert-receiving through-opening (38), and at least one insert-retaining surface (40) which is to be oriented upwards when the carrier (30) closes the tray (12);
- at least one insert (32) which is movably mounted relative to the carrier (30) between a culture position, in which the insert (32) is engaged through the receiving opening (38), and a handling position, in which the insert (32) is disposed away from the carrier (30),
the insert (32) comprising a bottom wall (68) carrying a culture surface (78) and a lateral wall (70) delimiting an upper opening (73), the insert (32) delimiting at least one passage (74) for feeding the culture surface (78) with culture medium, which passage is separate from the upper opening (73);
wherein the device (16) comprises releasable means (34) for retaining the or each insert (32) in its culture position, the releasable retaining means (34) being capable of holding the or each insert (32) in the receiving opening (38) when the carrier (30) and the or each insert (32) are conjointly turned over, which orients the retaining surface (40) downwards, and wherein the retaining means (34) form a bayonet-type mechanism.

2. Device (16) according to claim 1, **characterised in that**, in the culture position, at least one support surface (80, 86) of the insert (32) is applied to the retaining surface (40), the releasable retaining means (34) being capable of keeping the support surface (80, 86) of the insert (32) applied to the retaining surface (40) when the carrier (30) and the or each insert (32) are conjointly turned over, which orients the retaining surface (34) downwards.

3. Device (16) according to any one of the preceding claims, **characterised in that** the releasable retaining means (34) comprise a retaining member (100; 140) which is integral with a first of the insert carrier (30) and the insert (32), and a complementary retaining member (102; 142) which is integral with a second of the insert carrier (30) and the insert (32) in order to cooperate with the retaining member (100; 140) at least when the carrier (30) and the or each insert (32) are conjointly turned over.

4. Device (16) according to claim 3, **characterised in that** the retaining member (100) comprises a projection, the complementary retaining member (102) delimiting at least one groove (106) for grasping the projection, the size of which groove is adapted to hold the projection in the groove (106) by grasping.

5. Device (16) according to claim 3, **characterised in that** the retaining member comprises an abutment (140), the complementary retaining member comprising a complementary abutment (142), the insert (32) being displaceable in the receiving opening (38) between an inserted position, in which the space situated above the abutment (140) along an insertion axis (A-A') of the insert (32) into the receiving opening (38) is free, and the culture position, in which the complementary abutment (142) is disposed in the space situated above the abutment (140) along an insertion axis (A-A') of the insert (32) into the receiving opening (38).

6. Device (16) according to claim 5, **characterised in that** the insert (32) is rotatably mounted about the insertion axis (A-A') between the insertion position and the culture position, the releasable retaining means (34) advantageously comprising a surface (136) for blocking the rotation of the insert (32) in its locked culture position.

7. Device (16) according to any one of claims 3 to 6, **characterised in that** the insert (32) comprises at least one arm (66) which projects transversely outwards relative to the lateral wall (70), the arm (66) carrying one of the retaining member (100) and the complementary retaining member (102).

8. Device according to any one of the preceding claims, **characterised in that**, in the culture position, the insert (32) is fixed axially along the insertion axis (A-A').

9. Device (16) according to any one of the preceding claims, **characterised in that** the lateral wall (70) delimits at least one cut-out (76) which opens at the top, the cut-out (76) extending axially over a height which is greater than the thickness of the body (34).

10. Device (16) according to any one of the preceding claims, **characterised in that** the bottom wall (68) delimits a groove (150) for guiding a liquid, which groove extends radially towards the culture surface (72).

11. Device (16) according to any one of the preceding claims, **characterised in that** the insert (32) comprises a gripping member (84) which projects away from and above the carrier (30) when the insert (32) occupies its culture position.

12. Device (16) according to any one of the preceding claims, **characterised in that** the or each receiving opening (38) comprises a central region (50) capable of receiving the insert (32) in the culture position and a peripheral notch (52) extending radially away from the central region (50) in order to allow a tool to be inserted into the tray (12) through the carrier (30) when the insert (32) is received in the central region (50).

13. Cell or tissue culture assembly (10; 130), **characterised in that** it comprises:
- a tray (12) having at least one cavity (20) for receiving a culture medium (14);
- a device (16) according to any one of the preceding claims, the carrier (30) being mounted on the tray (12) in order to partially close the cavity (20).

14. Assembly (10; 130) according to claim 13, **characterised in that** the carrier (30) is mounted on the tray (12) in such a manner that a culture surface of a bottom wall (68) of an insert (32) is disposed horizontally.

15. Assembly (10; 130) according to either claim 13 or claim 14, **characterised in that** it comprises a cover (18) capable of covering the carrier (30), the carrier (30) and the or each insert (32) being received in a sterile manner between the tray (12) and the cover (18), in the same plane.
